# EUROPEAN PATENT SPECIFICATION

(11) **EP 0 604 546 B1**
(45) Date of publication and mention of the grant of the patent: **15.01.2003**
(21) Application number: 92920322.2
(22) Date of filing: 16.09.1992
(51) Int. Cl.: A61F 2/04, A61F 2/06

(54) **Extruded, controlled porosity implantable multi lumen device and method for making the same**
Implantierbare mehrlumige, eine kontollierte Porosität aufweisende, extrudierte Vorrichtung und Verfahren zur Herstellung
Dispositif implantable extrudé, à lumières multiples, à porosité régulée, et méthode de fabrication

(30) Priority: 16.09.1991 US 760716; 16.09.1991 US 760717; 16.09.1991 US 760718; 16.09.1991 US 760728; 16.09.1991 US 760753
(43) Date of publication of application: 06.07.1994
(60) Divisional application: 01200633.4
(73) Proprietor: ATRIUM MEDICAL CORPORATION, Hollis, NH 03049 (US)
(72) Inventor: HERWECK, Steve, A., Nashua, NH 03062 (US); KARWOSKI, Theodore, Hollis, NH 03049 (US); MARTAKOS, Paul, Pelham, NH 03076 (US)
(74) Representative: Greenwood, John David
(86) International application number: US9207828
(87) International publication number: WO93005730

(56) References cited:
- EP-A- 0 385 168
- EP-A- 0 406 665
- WO-A-88/06026
- WO-A-88/10103
- WO-A-91/08783
- DE-A- 3 924 663
- JP-A- 62 172 960
- SU-A- 904 693
- US-A- 2 978 787
- US-A- 3 516 408
- US-A- 3 805 301
- US-A- 4 110 246
- US-A- 4 202 349
- US-A- 4 309 776
- US-A- 4 416 028
- US-A- 4 619 641
- US-A- 4 755 171
- US-A- 4 790 313
- US-A- 4 973 609
- US-A- 5 002 661
- US-A- 5 024 232
- US-A- 5 024 671

## Description

### Background of the Invention

The present invention relates to implantable prostheses, and particularly to implantable prostheses having a passageway, or lumen for carrying a biological fluid. In its simplest form the invention may relate to a vessel and the biological fluid may be blood, but in other embodiments, the prosthesis may constitute a patch or graft of an organ, with the lumen defining an organ-related fluid path carrying some other biological fluid, such as an enzyme, hormone, secretion or waste product made or processed by the organ. In the prior art, a great deal of research and experimentation have been performed with vascular grafts to identify materials which have the necessary structural properties yet are compatible with cellular growth such that they may immediately function as blood vessels, yet provide a structural framework on which vessel tissue may regenerate. In various embodiments of the invention, as described more fully below, applicant describes a unique structure capable of supporting tissue growth, yet having a functional geometry including a lumen.

One type of implantable device is a synthetic vascular graft such as is commonly used to replace damaged or dysfunctional arterial or venous pathways, for example at the site of an aneurysm or occlusion. Bypass grafts are often used to divert blood flow around damaged regions to restore blood flow. Another use of vascular prostheses is for creating a bypass shunt between an artery and vein, specifically for multiple needle access, such as is required for hemodialysis treatments. Following multiple percutaneous invasions into a vein, the vein may either collapse along the puncture track or become aneurysmal, leaky or fill with clot, causing significant risk of pulmonary embolization. Vascular prostheses have been used for many years as an alternative to patients' own veins for vascular access during hemodialysis.

Materials research has led to the development of some synthetic materials for use in artificial vascular prostheses. For example, polytetrafluoroethylene (PTFE), a polymeric material which may be stretched to a specific length and expanded to a specific thickness, is often used to fabricate single lumen artificial veins and arteries. When thus stretched, or expanded, PTFE forms a network of interrelated nodes and fibrils. The diameters of the fibrils and internodal distances vary depending upon the conditions and rate at which the PTFE is stretched and/or expanded. Typical stretched and/or expanded PTFE articles have an internodal distance ranging from approximately twenty to approximately thirty microns.

An advantage of stretched and/or expanded PTFE is that the diameters of the fibrils can be made much smaller than the diameters of fibrils of knitted or woven fabrics which have previously been used for vascular prostheses. Moreover, due to the ability to control the pore diameter and porosity of PTFE tubing used, for example, for vascular prostheses, it is possible to decrease the occurrence of thrombosis associated therewith. Typically, however, PTFE vascular grafts cannot safely be used to withdraw blood until they have been in place in the body for a minimum of 14 days after surgery and have become surrounded by fibrotic tissue. This is because bleeding occurs at the site of a needle puncture in PTFE grafts if fibrotic tissue is absent. Complications which can result from early puncturing of PTFE arteriovenous fistulas include a hematoma surrounding the graft, false aneurysm, and graft occlusion.

Various other synthetic materials, in addition to PTFE, have been used for vascular grafts, including Dacron® brand and other synthetic polyester fibers, mandrel spun polyurethane, and silicon elastomer fibers. Additionally, vascular grafts have been formed using autologous saphenous vein, modified bovine carotid xenograft, and modified human umbilical vein. None, however, has overcome the problems associated with early failure of the graft following implantation.

In an effort to address these problems, various types of vascular access devices have been developed. One example of such a device is described by Tesio in U.S. Patent No. 4,898,669 (Feb. 6, 1990). This device is a catheter system mechanically coupled with a prosthetic vascular graft for use in blood purification. Devices available for long-term, repeated vascular access allow a catheter to be introduced to a blood flow pathway, but they do not generally allow chronic, permanent implantation. In some cases, due to the materials used, an autoimmune response occurs resulting in the formation of an occlusive hematoma.

Another vascular graft is disclosed in U.S. Patent No. 4,619,641 (Oct. 28, 1986) to Shanzer which discloses a coaxial double lumen device for use in hemoaccess. The space between the two lumena is filled with a self-sealing, non-biodegradable polymer which does not permit escaped bleeding following needle puncture. The Shanzer product consists of an outer tube positioned over an inner tube, both tubes being made of expanded PTFE.

While research has also led to the development of some improved drug delivery systems, these systems still require a significant amount of medication to achieve a therapeutic level for an organ specific function such as is required for a whole body systemic effect. Conventional drug delivery systems, for example, including orally applied tablets and liquids, injections, and localized intravenous infusions, all must be applied in rather large doses to achieve a systemic effect. Introduction of a catheter directly into a blood flow pathway, for example, results in delivery of bolus amounts of drug to a patient's system. This results in uncontrolled and often varied, whole body physiological effects, as well as blood vessel wall injury at the site of penetration. Much of the current research in drug delivery, therefore, has been directed to allowing delivery of drugs to a patient in a controlled, non-bolus manner, and in generally smaller doses. This requires that delivery be localized, or targeted, to a cell specific organ.

Presently, material research has also enabled modern developments such as transdermal and time-release delivery systems which also create a whole body systemic effect. Future research suggests the extended use of chemically modified drugs complexed to carrier or bioactive agents, organic vesicles, and controlled delivery systems such as micro-pumps and mini-pumps. These and other new drug delivery systems are discussed, for example, by R. Langer, In: "New Methods of Drug Delivery", Science 249:1527-1533 (September 28, 1990).

Additionally, recently published evidence indicating that cellular activities are controlled by receptors, "molecular switches" on the membrane surface of cells suggests that bioactive and pharmaceutical drug interactions, whether initiators or inhibitors, can be utilized to improve implantable organ and autogenous organ transplant performance. These receptors control cellular activities by binding with highly specific substances referred to as "ligands." Like the action of a key in a lock, ligands fit into receptors and, if the fit is precise, turn on or off certain cellular processes. Some ligands act as antagonists to inhibit cellular activities by blocking a receptor. Research has shown that many aspects of cardiovascular disease are controlled by specific cell surface receptors.

Another problem associated with known vascular grafts stems from their delicate structure making them difficult to handle and position properly during surgery. Due to their circumferentially uniform appearance, the grafts may be twisted during implantation, which can reduce the openness, or patency, of the implanted graft.

Another problem associated with known vascular grafts is that they are transparent to known non-surgical techniques for viewing that are generally used to detect structures in the body. These techniques include x-rays, MRI scanning, fluoroscopy, ultrasound, and nuclear magnetic resonance. As a result, post-implantation examination of known vascular grafts is difficult. If a surgeon suspects that a blockage of an implanted vascular graft has occurred, for example, he must inject radiopaque dye into the patient through the graft. The dye allows the graft to be visible during fluoroscopic examination to determine whether it has collapsed or is satisfactorily transporting blood flow. This process is invasive to the patient and places further burdens on the patient's circulatory system.

Various marking devices such as metal tabs which are sutured to a prosthesis, have been developed to avoid the need for injecting a radioopaque dye into a patient's bloodstream. However, there remains a need for an improved system of indicating the patency of a graft vessel.

Other problems associated with vascular grafts formed of known materials and configurations are that their biocompatibility, non-thrombogenic potential, cell harboring, and seeding properties are limited. Specifically, for example, intimal hyperplasia, which is a naturally occurring phenomenon characterized by progressive cellular closure of a blood vessel lumen, threatens the patency of almost all known vascular graft material. Even when surgically repaired or exposed to less intensive manipulative techniques such as balloon dilation, mechanical dilation, laser ablation or mechanical dissection by anthrectomy, intimal hyperplasia is the primary cause of stenosis of all implantable vascular grafts and restenosis of natural arteries following repair of diseased blood vessels.

One cause of intimal hyperplasia is the proliferation of smooth muscle cells into the lumen of the graft. Other causes include injury to the venous system and/or arterial circulation network, caused by trauma, disease, or systemic factors such as hypercholesterolemia.

While intimal hyperplasia is known to cause significant lumenal obstruction of vascular grafts, the detailed cellular mechanisms leading to smooth muscle cell proliferation are not completely understood. It is believed, however, that growth factors such as platelet derived growth factor (PDGF) initiate a number of intracellular events, called "regulatory signals." These signals include the activation of protein kinase C. Additionally, different growth-stimulating factors are thought to initiate different signals. These different signals lead to a set of common pathways which stimulate DNA synthesis. Such pathways are called obligatory events. It is not clear which of these obligatory events, though, are responsible for intimal hyperplasia.

Also, lumenal blood vessel injury, whether micro-capillary (0.5 mm or less diameter) or aortic (up to 30 mm diameter), induced by trauma, such as mechanical stress, or progressive disease states such as arteriosclerosis or mechanical hemodynamic stress, causes activation of platelets, injury and necrosis of smooth muscle and endothelial cells, and resultant leukocyte infiltration. These events result in the production and release of factors that stimulate smooth muscle cell migration and proliferation from adjacent tissue, subsequently leading to intimal hyperplasia. As stated, such growth, when induced by trauma or progressive disease, without pharmacologic intervention, causes stenotic closure and failure of most autogenous organ transplantations such as coronary artery bypass grafting and synthetic implantable vascular graft devices.

Known treatments of intimal hyperplasia involve the administration of various drugs that inhibit muscle cell proliferation. For example, somatostatin inhibits tumor cell growth. Angiopeptin, a synthetic peptide analog of somatostatin, reduces myointimal proliferation. Trapidil, an antianginal agent possessing vasodilatory and antiplatelet properties, and Terbinafine, an antifungal agent, both are effective antiproliferative agents. Colchicine, a drug which possesses antimitotic and antisecretory properties, also is effective in reducing myointimal thickening.

Besides antiproliferative agents, drugs that inhibit the synthesis and secretion of extracellular matrix are also useful, since a large proportion of the restenotic tissue is composed of extracellular matrix. Because smooth muscle cell migration is an essential step in intimal proliferation, agents that inhibit SMC migration ultimately inhibit proliferation.

Other techniques have been developed, for improving the patency of implantable devices. One such process is glow discharge polymerization as taught by United States Patent 4,632,842 to Karwoski et al. Karwoski teaches coating, by the use of glow discharge conducted in a tubular reaction vessel, the surface of an elongate organic substrate with substantially uniform, very low surface-energy coating. Still, even with the Karwoski teaching, the proliferation of smooth muscle cells into known . prosthetic vascular grafts is not controlled or reduced sufficiently to prevent intimal hyperplasia

Another problem associated with known prosthetic devices arises in connection with the use of such devices as a means for drug delivery. That is, while it is known that various prostheses can be coated with bioactive and pharmaceutic agents for blood contact, the limited blood contact surface area within the single lumen of known prostheses used for this purpose limits the amount of agent that can be effectively distributed into the flow through body fluid. Additionally, the high ratio of blood flow-through to contact surface area results in a high level of wash-off.

In addition to the foregoing limitations of the prior art, certain inconveniences or shortcomings bear further note.

During prosthetic surgery, for example, in the course of replacing or by-passing damaged arteries and veins, the need often arises to have prosthetic devices of different diameters. In the physical setting, arteries are generally smaller in diameter than veins, thus requiring arterial and venous grafts to be available in a range of diameters. One way to provide grafts of different diameters to the surgeon at the time of implantation is to provide an array of individual vascular grafts each having a different diameter to the surgeon. These grafts are of a fine material, which can become entangled or torn during handling. In order to make these individual grafts communicate with each other, they must be sutured together.

WO 91/08783 discloses an implantable device for administering drugs or other substances to body tissues of a patient is disclosed. The device is designed to allow controlled ingrowth of connective tissue into the device, and a solution may be pumped or infected into the device where the solution is diffused through the ingrown connective tissue and out into the body. The device includes an outer casing defining an inner space, said casing being formed of porous synthetic material capable of allowing body tissue to grow through the pores of the synthetic material into the inner space (42) and an inlet catheter of non-porous synthetic material in communication with said inner space for conveyance of substances thereto.

US-A-5,024,671 discloses an implantable prosthetic device having the features of the preamble of claim 1. The present invention, in a first aspect, is such a device characterized by the features of the characterizing portion of claim 1. The invention according to a second aspect of the present invention is a method of making an implantable prosthetic device as claimed in claim 10.

The invention features an implantable biocompatible device formed or a material of controlled porosity and having a primary lumen extending therethrough, and at least one secondary lumen, which as discussed further below, may according to different aspects be filled with materials, or may itself constitute another primary lumen.

The invention features an implantable, biocompatible prosthetic device for the sustained release of a drug or other bioactive material directly into a blood or other body fluid flow path. The device is a polymeric bi- or multilumenal tubular article which can be attached, for example, to an artery or vein to form a vascular graft or shunt. The device can also be used to provide organ to organ fluid communication. The device contains a primary lumen, which is dedicated to the flow of blood or other body fluid and at least one secondary lumen. The lumena are separated by a microporous, semi-permeable wall which permits passage of an agent from the secondary lumen to the primary lumen. Additionally, the microporosity of the separating wall promotes the growth, motility, and/or migration of cells into and through the wall.

The secondary lumen is particularly well suited to contain a material, such as a drug or other bioactive agent, which permeates the porous wall between the secondary lumen and the primary lumen over a sustained period of time. The rate at which the drug or other agent penetrates the porous wall is determined by several factors, including the size and number of the pores and the size of the drug molecule.

In one embodiment of this aspect of the invention, the prosthetic device comprises a tube which is adapted for attachment to a blood flow pathway, and for conducting the flow of blood therethrough. The tube has a biocompatible or bioinert exterior surface, and defines a primary lumen axially extending along the length of the tube, and at least one additional or secondary lumen. The secondary lumen is separated from the first lumen by a microporous wall which allows a drug introduced into the secondary lumen to diffuse across the wall and into the primary lumen, and thus directly into the blood flow pathway. This is caused by patient cells penetrating the exterior wall of the tube and displacing air contained in the micropores of the tube's microporous structure. The displaced air, in turn, displaces material contained in the secondary lumen forcing the material to diffuse into the primary lumen.

The secondary lumena can be pre-filled with a selected drug which time-diffuses or otherwise perfuses across the membrane. The lumena can also be filled with drug-producing cells which disperse a drug product into the secondary lumena, from which it then diffuses, as discussed above, into the primary lumen. The primary lumen and/or the secondary lumena can also be seeded with cells, for example endothelial cells, which proliferate at the site of attachment due to the cell to cell contact afforded through the walls of the microporous structure. The invention allows the endothelial cells to thrive, therefore, which helps to prevent undesired occlusion of the graft.

In another embodiment of this aspect of the invention, the tube features an external drug delivery device attached thereto by a second tube or catheter. The catheter is connected to the secondary lumen. The delivery device injects the drug into the secondary lumen from an external source. The device can be any of a variety of commercially and technologically available systems, such as, for example, a biologically activated mini-pump which is either subcutaneously or extracutaneously located, or an external mechanical pump.

The present prosthetic device is preferably made from stretched and expanded polytetrafluoroethylene (PTFE). Stretched and expanded PTFE contains a porous network of nodes and fibrils which are created during the stretching and expansion process of porous tubing from PTFE. This porous network provides a semi-permeable wall or membrane between the lumena of the device.

Embodiments of the invention may be used in a method for delivering directly into a fluid flow pathway in a controlled manner a bioactive material, such as a prophylactic or therapeutic drug, or a diagnostic material, such as a radiolabeled antibody. This may include the steps of pre-filling one of the secondary lumena of the above-described implantable device with a bioactive material and implanting the device in a fluid flow pathway such as a vein or artery. In this manner, fluid flow is established through the primary lumen of the device. The pre-filled material diffuses across the wall or membrane separating the secondary lumena from the primary lumen in a controlled manner, thereby delivering the drug directly into the fluid, blood or otherwise, flowing through the primary lumen. The lumena may be prefilled by inserting a solid wire or rod of bioactive material releasably bound in a resorbable binder material.

A separate external drug delivery system may be attached to at least one of the secondary lumena. This allows transport of the drug into the secondary lumen from an external source. The drug then diffuses across the interlumenal wall as described above.

The invention has several advantages. Its use can allow implantable vascular grafting and controlled and/or continuous drug delivery to be combined. Additionally, use of the invention can allow a bioactive substance to be delivered directly into a patient's bloodstream at a controlled rate without using intravenous injection, which generally must be performed in a hospital or doctor's office. Embodiments of the device allow a bioactive substance to be injected into the secondary lumen all at once from an external source, then released into the patient's bloodstream at a slower, continuous rate as the substance passes from the secondary lumen into the primary lumen of the graft. This sustained release of a substance into the bloodstream over time is less likely than known drug delivery methods to result in distal embolization. The graft provides a site for repeated cannulation which does not require directly accessing the bloodstream and therefore reduces the incidence of bleeding at the injection site.

Various extrudable materials are suitable for forming the implantable body of the invention. In particular, expanded polytetrafluoroethylene (PTFE) has been found to be well suited for the present invention. When using PTFE for this purpose, as described in greater detail herein below, a paste of the material is extruded in the basic form of the implantable device which is then expanded at a specified rate to create an interrelated network of nodes and fibrils.

### Brief Description of the Drawings

The foregoing and other objects of this invention, the various features thereof, as well as the invention itself, may be more fully understood from the following detailed description, when read together with the accompanying drawings, in which:
FIGURE 1A is a schematic perspective view of a bilumenal vascular prosthesis of the present invention.
FIGURE 1B is a schematic longitudinal cross-sectional view of the embodiment of FIGURE 1A.
FIGURE 1C, 1D and 1E show schematic cross-sectional views of alternative configurations of the bilumenal device of FIGURE 1A.
FIGURE 2 shows a schematic front elevation view of a die used in the manufacture of the bilumenal device of FIGURE 1A.
FIGURE 2A and 2B show schematic cross-sectional views of the die of FIGURE 2 taken along planes A-A an B-B, respectively.
FIGURE 3 is a schematic side elevation view of a bilumenal device in which the secondary lumen is partially co-extensive with the primary lumen.
FIGURE 3A is a schematic cross-sectional view of the embodiment of FIGURE 3.
FIGURE 4 is a schematic perspective view of a multilumenal device of the invention.
FIGURE 5 is a schematic longitudinal cross-sectional view of an embodiment of the invention having a preattached mini-pump.
FIGURE 6A is a schematic perspective view of an alternate multilumenal embodiment of the invention.
FIGURE 6B is a schematic perspective view of an alternate configuration of the embodiment of FIGURE 6A.
FIGURE 7 is a schematic perspective view of an alternate configuration of the multilumenal embodiment of FIGURE 6A.

Like reference characters in the respective FIGURES indicate corresponding parts.

### Detailed Description of the Invention

In its broadest aspect, the invention features a multi-lumenal prosthetic device for implantation into a patient. The invention can be utilized, for example, as a vascular graft providing sustained release of a selected bioactive agent or diagnostic material directly into a blood or other fluid flow pathway. The device has at least two lumena which are separated by a porous, semi-permeable wall. In the case of the device being used as a vascular graft, it is grafted onto a vein or artery in an individual such that the primary lumen becomes part of the individual's blood flow pathway. The secondary lumen is filled with a material such as, for example, a bioactive or diagnostic agent and the porous wall between the lumena allows the material disposed in the secondary lumen to diffuse into the bloodstream flowing through the primary lumen. As described in further detail below, the device allows the release of the material across the wall or membrane into the bloodflow pathway in a controlled manner.

The device comprises a main, or primary lumen, which is of a diameter sufficient to allow blood flow appropriate for the artery or vein to which it is attached to occur. Thus, the device has the geometric configuration of a tube, open at least at one end, and typically at both ends. The open end is sutured to an opening in the patient's arteriovenous pathway, thus becoming an extension of that pathway.

The device also contains at least one secondary lumen adjacent to the first lumen. At least one of the secondary lumena contains or is adapted to contain the selected bioactive or diagnostic materials. These materials can include, for example, therapeutic or prophylactic agents, such as a drug, protein, enzyme, antibody or other agent, or cells which produce a drug, protein, enzyme, antibody, or other agent. The diagnostic material can include, for example, a radiolabeled antibody or antigen.

In one embodiment of the invention, one or more of the secondary lumena are pre-filled with the bioactive material. For example, the pre-filled secondary lumen can contain cells which secrete a bioactive agent. As a variation of this embodiment, another embodiment may include a pre-filled lumen in conjunction with other secondary lumena into which drugs or diagnostic materials are introduced after implantation.

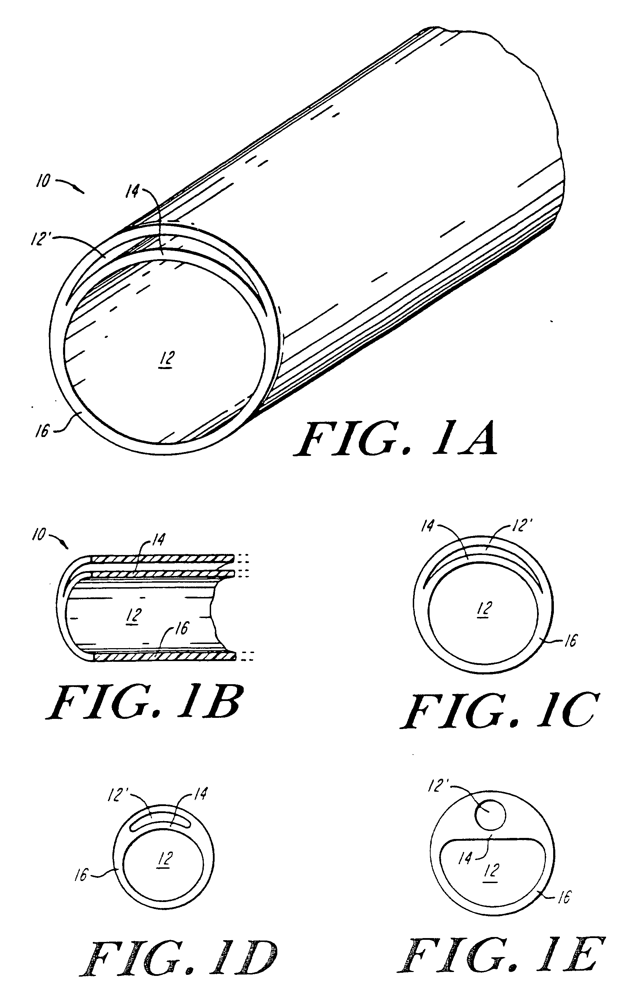

Specific embodiments of the device are illustrated by the Figures. FIGURE 1A shows a bilumenal tubular structure 10 having a first lumen 12 and a secondary lumen 12'. The first lumen 12 has a structure sufficient for blood flow therethrough. For a vascular graft, the diameter of this lumen 12 is generally the same or similar in size to the host artery or vein to which it is grafted. As mentioned, however, the structure 10 can be formed to accommodate other types of fluid flow.

As shown in FIGURE 1B, the secondary lumen 12' is adjacent the first lumen 12, and is separated by a semi-permeable, micro-porous, wall 14. The wall 14 has a permeability sufficient to allow diffusion of the bioactive agents or diagnostic material of choice from the secondary lumen 12' into the fluid flow pathway defined by the first lumen 12. In a multi-lumen arrangement, i.e., a device having more than two lumena, the adjoining wall 14 lies in communication between each of the secondary lumena and the primary lumen. Alternatively, there can be a semi-permeable wall between each secondary lumen 12' and the first lumen 12, with an impermeable wall among the secondary lumena.

The thickness and permeability of the wall 14 can be adapted to accommodate different drugs, bioactive or bioinert agents, and the like, and to control the rate at which the material disposed in the secondary lumena 12' diffuses across the wall 14. Control over the release of the drug or agent can be obtained by choosing appropriate molecular weights, degrees of crystallinity and/or expansion parameters in the polymer matrix forming the structure 10. The wall 14 can be manufactured having a predetermined permeability factor, and the concentration of materials to be transported across the membrane can be selected accordingly.

The opportunity to control the permeability of the wall 14 is afforded during the extrusion and expansion process as discussed in greater detail below. By controlling the intermodal distance of the polymeric matrix and properly selecting the specific resin composition and expansion conditions, the porosity of the wall 14 can be determined.

The outer diameter (OD) of the external wall of the structure 10 is generally in the range of from about 3 mm to about 30 mm. The internal diameter (ID) of the primary lumen is generally from about lmm to about 28 mm depending upon the type of blood flow through the prosthesis. The OD and ID vary according to the type of pathway for which the prosthesis is used. For example, arterial prostheses will generally have OD of from about 6 mm to about 18 mm, whereas venous prostheses will generally have an OD of from about 12 mm to about 24 mm. The ID of the secondary lumena 12' is generally from about .1 mm to about 6 mm, depending upon the OD.

As shown in FIGURES 1C, 1D, and 1E, the cross-sectional configuration of the lumena designed in accordance with the invention can vary in size and shape depending upon the specific application. In FIGURE 1C, the secondary lumen 12' extends over approximately one-third of the circumference of the lumen 12. FIGURE 1D shows a diminished relative size of the secondary lumen 12' with respect to the first lumen 12, and FIGURE 1E shows a first lumen 12 having an adjacent secondary lumen 12' of dimensions sufficient only to transport substances of a few microns in size. The relative size of the secondary lumen 12' depends on the type of substance to be transported through secondary lumen 12', and the desired rate of diffusion across wall 14. The permeability of wall 14 may be a factor in determining the appropriate size of secondary lumen 12'.

The invention may be used in a method for delivering a bioactive agent or diagnostic material directly to a patient's body fluid, bloodstream or otherwise, in a controlled manner. In such a method, a surgeon or other qualified person, surgically exposes the desired region of the patient for introduction of the prosthetic device 10. The desired site may, for example, be an area of occlusion or weakness in the patient's arteriovascular system. In such a case, during interruption of the patient's blood flow the prosthesis 10 is surgically implanted and sutured or otherwise secured in place. Proper positioning of the prosthesis 10 requires alignment of the primary lumen 12 with the blood flow pathway such that the blood flow is diverted through the primary lumen 12. The secondary lumen 12' either contains or is filled with a drug or agent of choice. The drug or agent perfuses across the interlumenal wall 14 into the bloodstream at a controlled and substantially continuous rate. In this manner, the use of the present invention can provide continuous administration of the drug over a prolonged period of time similarly to controlled release systems which deliver a drug at a predetermined rate over a definite period of time.

In an example of use of the present invention, an antibody which is specific to a protein indicative of the presence of malignancy is introduced into the vascular system of a patient utilizing the present device and method. The device 10 is surgically implanted in a patient's vascular system and blood flow is established through the primary lumen 12. The antibody is added to the secondary lumen 12' either before implantation by pre-filling the lumen or after implantation by injecting the antibody composition into the secondary lumen. The antibody is labelled, for example, with a remotely detectable radioisotope such as ¹²⁵I. The labelled antibody moves across the porous wall 14 between the primary lumen 12 and the secondary lumena 12' into the patient's blood flow pathway, which flows through the primary lumen 12. Once in the pathway, the labelled antibodies travel to the site of the malignancy and bind at the appropriate protein binding sites. The concentration of the radioisotope at the target site can then be detected using an appropriate detection device, e.g., a gamma camera. Additional doses of the antibody composition can be injected into the secondary lumen 12' through a cannula, or through a pre-attached catheter which communicates with the secondary lumen 12', without directly invading the patient's bloodstream.

In another exemplary use of the present invention, combinations of anti-coagulant platelets and plasminogen activators, such as heparin, hirudin and tPA can be injected into the secondary lumen 12' to be released at a controlled, continuous, rate into the bloodstream. Continuous release of a solution containing either heparin, tPA, or combinations of the two into a vascular graft area has been shown to be effective in reducing occlusion from intimal hyperplasia, while not resulting in whole body systemic anti-coagulation. Clifton et al., Heart & Lung, 199(1): 115-118 (March, 1991).

In another example of use of the invention, the prosthesis 10 can be used for distribution of chemotherapy agents which are often very toxic and often cause arterial and venous blood vessel destruction at the needle catheter entry site. A major complication of such chemotherapy is the constant risk of permanent, significant delivery site complications such as infection or occlusion due to thrombus formation at the vessel wall injury. The high concentration of such toxic agents very often has a deleterious effect on the blood vessel, further complicating the healing required for the needle tract or catheter injury incurred at the site of drug infusion.

The device 10 eliminates such needle and catheter injury to the blood vessel and large bolus chemical exposure to the native vessel with these highly toxic chemotherapy agents. Moreover, the device 10 can be replaced if long term therapy causes eventual failure or thrombus occlusion. A vein or artery permanently destroyed by chronic therapy, on the other hand, is irreplaceable. Similar problems and corresponding solutions also exist for hemodialysis patients whose native veins are consumed by repetitive needle penetration and permanent blood vessel wall injury.

The secondary lumen forming a chamber or reservoir for a drug or other bioactive substance need not be fluid-filled, but may be filled with a solid or powder, which,upon perfusive exposure to surrounding fluids, releases its active agent into the fluids. In one aspect of the invention, a substance is incorporated in a binder that is drawn or extruded to form a wire or rod of active materials, which is then cut to length and threaded into the secondary lumen. Perfusion rate and dose are carefully controlled by selection of a binder material having the desired in vivo solubility and of a wire gauge, selected to determine a desired total amount of exposed surface area. In addition, the solid wire or rod is cut to length, to determine the total dose. The provision of medications in a leachable or resorbable wire body in this manner is thus seen to offer great simplifications over the prior art techniques of medication or coating a prostheses, which generally involved lengthy steps of soaking and conditioning the graft material.

A method of using this sevice includes providing a vessel or organ graft prosthesis surgically implanted and spliced into a vessel or organ, the graft prosthesis having one or more included chambers in the form of lumena extending along the graft, and providing one or more string, rod or wire shaped solid pieces of a treatment material in one or more of the lumena to locally perfuse the material into the graft over an extended time.

The tube structures of the invention can be manufactured from any suitable biocompatible material that can be arranged to form a microporous structure. Polymeric materials which are useful for this purpose include, for example, either expanded or unexpanded polytetrafluoroethylene (PTFE), Dacron® brand polyester, and other synthetic polyester fibers such as mandrel spun polyurethane and silicone elastomeric fibers. Also, copolymeric materials such as described in U.S. Patent Nos. 4,187,390 and 4,973,609 can be utilized. These are materials made up of more than one type of monomer and have advantages as described in the cited patents, in some applications. The structures can also be formed by extrusion, form molding, or weaving using techniques well known in the art.

In a preferred embodiment, the inventive prosthesis is manufactured by paste forming and rapidly stretching and/or expanding highly crystalline, unsintered, polytetrafluoroethylene. Paste forming by extrusion of PTFE is well-known in the art. Generally, the steps in paste-forming include mixing the resin with a lubricant, such as odorless mineral spirits, and then forming the resin by extrusion into shaped articles. The lubricant is removed from the extruded article by drying, following which the article is sintered by its being heated above its crystalline melting point of approximately 327°C. The sintered, unexpanded, article is a relatively impermeable product. To achieve a greater degree of permeability in the finished product, however, the prostheses of the invention can be formed from an unsintered resin.

Paste-formed, dried, unsintered, shapes can be further treated by expanding and/or stretching them in one or more directions under certain conditions so that they become porous yet retain their strength. Such stretching and expansion with increased strength occurs with certain preferred tetrafluoroethylene resins, e.g., PTFE. The porosity of the material is affected by the temperature and rate at which it is stretched and expanded. A method for manufacturing porous PTFE tubing appropriate for use in the present invention is described in detail, for example, in U.S. patent 3,953,566, and U.S. patent 4,973,609.

Stretched and expanded PTFE is characterized by a microstructure of nodes interconnected by small fibrils. The space between the nodes and the number of fibrils is controlled by changes in the temperature and rate of stretching and expansion of the PTFE, to produce tubing having predetermined porosity and flex qualities. For example, products which are stretched and expanded at high temperatures and high rates have a more homogeneous structure, i.e., they have smaller, more closely spaced nodes, which nodes are interconnected with a greater number of fibrils. While the resulting structure is stronger than products stretched and expanded at lower temperatures and rates, the porosity is also reduced. Thus, by controlling these two factors, it is possible to construct a series of tube structures having a range of porosity within a desirable range of strength.

Tube structures manufactured as described above begin to lose their crystallites and the crystallinity decreases above this temperature. This is accompanied by a concomitant increase in the amorphous content of the polymer. Amorphous regions within the crystalline structure greatly inhibit slippage along the crystalline axis of the crystallite and lock fibrils and crystallites so that they resist slippage under stress. Heat treatment may be considered to be, therefore, an amorphous locking process, which results in an increase in the amorphous content and of the strength of the treated structure. Heat treatment above 327°C has been found to cause a two-fold increase in the strength of PTFE is approximately 345°C, heat treatment is even more effective. Similar results can be achieved at lower temperatures if expose time is accordingly increased. The optimum heat treating temperature is generally in the range of from about 350°C to about 370°C, with heating periods in the range of from about five seconds to about one hour. Other factors upon which the strength the polymer matrix is dependent upon include the strength of the extruded material before expansion, the degree of crystallinity of the polymer, the rate and temperature at which the expansion is performed, and amorphous locking.

The tube structures of the invention can be formed using other paste-forming operations known to those skilled in the art, for example, any of the available molding processes. Resins other than PTFE may also be used which are generally formable into such tube structures, and which may result in relatively fluid impermeable structures.

The prosthesis 10 can be coated on both the interior lumenal and/or the exterior surfaces with a biocompatible material to render it more hydrophilic or hydrophobic, or to allow specific protein binding or attachment after implantation. Coating materials which are useful for this purpose include, for example, various glycoproteins, albumin or polymeric coatings often used for plasma polymerization, and solvable polymeric coatings such as EVA and PVA Due to the physiological properties of both the arterial and venous system, however, it is important for the prostheses 10 to be gas permeable, or selectively gas permeable, to permit oxygen-carbon dioxide exchange. However, even gas impermeable tube structures may be useful as vascular grafts in certain anatomical applications.

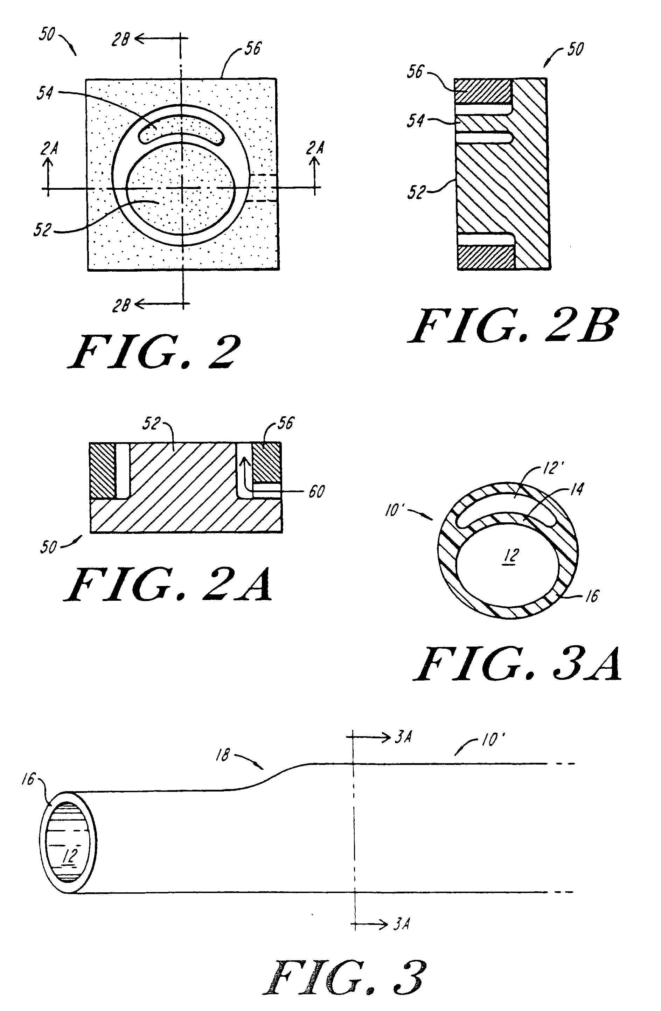

As stated, in the preferred embodiment, the prostheses of the present invention are formed by extrusion of PTFE which is performed using dies of predetermined shape of the type known in the art. FIGURES 1C through 1E, for example, show cross-sectional views of prostheses of the invention made using different exemplary dies. In particular, FIGURE 2 schematically shows an exemplary die 50, corresponding to the illustrated prosthesis of FIGURE 1A. The dies are manufactured from materials and according to methods which are well known in the art.

Generally, and as is illustrated in FIGURE 2, the die 50 consists of a peripheral support structure 56 encasing a first solid die-piece 52 for forming a first lumen, and a second solid die-piece 54 proximal to the first die-piece 52 for forming a secondary lumen. The specific spacing of the first die-piece 52 from the second die-piece 54 depends on the specific desired prosthesis configuration. As best shown in cross-section in FIGURE 2A, the die 50 may include an external port 60 for introduction of PTFE paste or the like for extrusion. FIGURE 2B shows in cross-section the exemplary die 50 of FIGURE 2, showing aperture 58 for forming membrane (14 of FIGURE 1) of the invention.

After the PTFE resin is formed, such as by extrusion as discussed above, it is stretched and/or expanded and then sintered while being held in the stretched and/or expanded state. Stretching refers to elongation of formed resin while expansion refers to enlargement of the formed resin perpendicularly to its longitudinal axis. The rate of stretching and the stretch ratio affect the porosity of the finished product in a predictable manner allowing a prosthetic device to be produced having a specified porosity. The rate of stretching refers to the percentage of elongation per second that the resin is stretched while the stretch ratio refers to the relationship between the final length of the stretched resin and the initial length of the stretched resin. For example, stretching an extruded PTFE tube at a stretch ratio of two to one and a stretch rate of sixty results in a porosity of approximately forty. This porosity is unitless and is determined as set forth on page eighty-four of the American Society For Testing of Materials' Special Technical Publication Number 898. So, for example, based on stretch ratios ranging from two to one, to six to one, a stretch rate of sixty percent per second yields a porosity of between approximately forty and approximately ninety, a stretch rate of one hundred and forty percent per second yields a porosity of between approximately sixty and approximately eighty-five, and a stretch rate of nine hundred percent per second yields a porosity of between approximately sixty-five and approximately eighty-five.

In addition to the porosity, the geometry of the node and fibril network of PTFE can be controlled during stretching and expansion. In the case of uniaxial stretching, that is, elongation of the formed PTFE resin along the direction of extrusion, the nodes are elongated causing the longer axis of each node to be oriented perpendicularly to the direction of stretch. Accordingly, the fibrils are oriented parallel to the direction of stretch. Biaxial stretching, additionally includes expanding the PTFE resin in the radial direction and can be utilized to produce a prosthetic device having a composite porosity. As in uniaxial stretching, the rate and ratio of radial expansion affects the resulting porosity of the prosthetic device.

In one embodiment, the apparatus of the present invention includes co-extruded plural lumena, the secondary lumena 12' of which extend substantially along the entire length of the first lumen 12. In another embodiment of the invention, however, as illustrated in FIGURE 3, the secondary lumen 12' extends along only a portion of the first lumen 12.

The illustrated prosthesis 10' of FIGURE 3 is structurally similar to the prostheses 10 described above. As shown in cross-section FIGURE 3A, the portion of the prosthesis 10' which includes a secondary lumen comprises a microporous, semi-permeable, wall 14 separating the secondary lumen 12' with the first lumen 12. However, in this illustrated embodiment, the secondary lumen only extends along a predetermined portion of the prosthesis 10'.

The embodiment shown in FIGURE 3 may be manufactured in a manner similar to that described above. The extrusion die for forming the partially-extending lumen may be modified in a manner known to those skilled in the art. For example, the die used in the extrusion of this embodiment of the invention may include a gate-type device which enables selective opening and closing of an aperture to coextrude a secondary lumen.

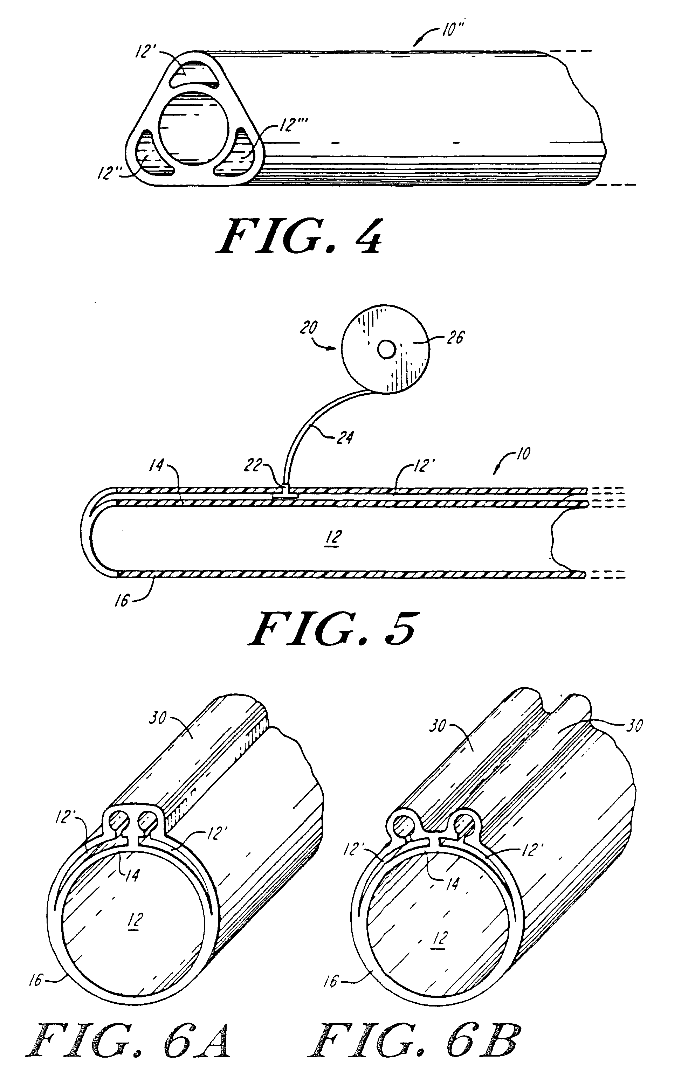

An alternative embodiment of the prosthesis of the present invention is illustrated in FIGURE 4. In the illustrated prosthesis 10', the first lumen 12 is essentially round, with the secondary lumen 12' forming a polygonal configuration around the first lumen 12. While this illustrated embodiment shows three secondary lumen 12', other forms of the invention may include fewer or more secondary lumena. In addition, the apparatus of the invention may have various overall geometric configurations, depending upon the anatomical destination of the prosthesis 10'.

An important aspect of the invention is the introduction of a bioactive agent, pharmaceutical, chemotherapy agent or diagnostic material into a secondary lumen for perfusion into the blood flow pathway of the patient as it is channeled through the first lumen of the prosthesis. This introduction of material may occur by including a mini-pump attached to the secondary lumen of the prosthesis, for example, by a catheter.

As shown in FIGURE 5, a mini-pump 20 may be placed in communication with at least one of the secondary lumen 12' for perfusion of a drug into the secondary lumen 12'. The mini-pump 20 can be used to deliver a predetermined amount of a drug at a preselected rate to the secondary lumen 12. Thereafter, the drug perfuses across the microporous, semi-permeable, wall 14 and into the bloodflow pathway of the first lumen 12. Mini-pumps which may be used with the prosthesis of the invention are commercially available. Some examples are Alza Pump, Thermedics' Infusaid Pump, Medtronic's Infusable Pump, and INFU·DISKS™ (Electrochemical Drug Delivery, Inc. San Diego, California) designed for subcutaneous delivery of drugs or diagnostic agents. The mini-pump 20 may be located externally to the patient, or may be surgically subcutaneously implanted.

As shown in FIGURE 5, the mini-pump 20 contains a drug reservoir 26 from which a connector tube 24 extends. The connector tube 24 may either be integral with the reservoir 26, or mechanically, detachably connected to the reservoir 26. The connector tube 24 extends from the reservoir 26 to a secondary lumen 12' of a prosthesis 10. The connector tube 24 may connect to the secondary lumen 12' by means of a mechanical attachment device 22, as illustrated, or may be formed integral with the secondary lumen 12'. Mechanical attachment devices are well known in the art, and include luer-locks. It is generally preferable that the mechanical attachment device is such that the mini-pump may readily be replaceable, thus attachment devices which do not require the application of pressure are preferable over pressure-lock devices.

Similarly, a mechanical pump, infusion system, or other drug delivery system located outside the host body may be attached for the delivery of a drug or other bioactive material into a secondary lumen. The drug delivery source may either be integral with the secondary lumen, or may be mechanically, releasably attached.

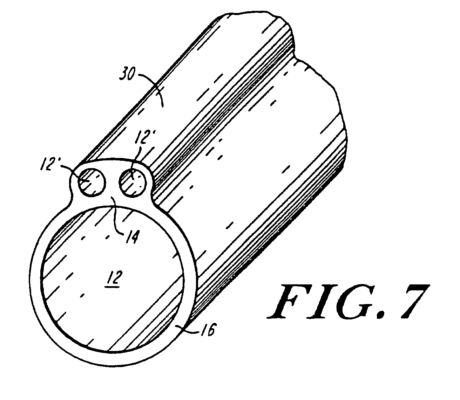

Alternate embodiments of the present invention are shown in FIGURES 6A and 6B. In those embodiments, the secondary lumena 12' form a raised platform 30 to facilitate subcutaneous needle access. Reservoirs 32 extend from the secondary lumena 12' along the sides of the first lumen 12 to provide a greater surface area for diffusion of the drug across the microporous, semi-permeable, wall 14. As shown in FIGURE 6B, needle access ports 34 may be separated for extracutaneous identification of the individual secondary lumena. In this manner, separate drugs may be delivered to separate secondary lumena.

Another embodiment of the prosthesis of the present invention is shown in FIGURE 7, wherein needle access ports 34 are located in a single raised platform 30. In this illustrated embodiment, the secondary lumena 12' do not include reservoirs that extend along the sides of the first lumen 12. The secondary lumena of the illustrated embodiment extend either partially or fully along the length of the first lumen.

As noted above, the prosthesis of the invention can be manufactured from any suitable biocompatible material, such as PTFE, Dacron®, or other synthetic polyester, or mandril spun polyurethane or silicone elastomer micro-fibers that can be arranged to form a microporous structure. Hybrid constructions of these same materials are suitable as well. Also, copolymeric materials such as described in U.S. Patent Nos. 4,187,390 and 4,973,609 can be utilized. These are materials made up of more than one type of monomer and have advantages, in various applications, described in the In a preferred embodiment, described in further detail below, the implantable device is manufactured from stretched and/or expanded PTFE tubing by rapidly stretching highly crystalline unsintered polytetra-fluoroethylene in one or more planes or axes.

Stretched and expanded PTFE is characterized by a microstructure of large nodes interconnected by fibrils, with the space between the nodes, internodal distance, and the number of fibrils controlled by changing the temperature and rate of expansion of the PTFE to produce structures having predetermined porosity and flex qualities. Internodal distances of from smaller than approximately 0.5 microns to as large as approximately 60 microns are suitable for use with the present invention.

Products which are expanded at high temperatures and high rates have a more homogeneous structure, i.e., they have smaller, more closely spaced nodes, which nodes are interconnected with a greater number of fibrils. While the resulting structure is stronger than products expanded at lower temperatures and rates, the porosity is also reduced. Thus, by controlling the two factors, it is possible to construct a series of tube structures having a range of porosity within a desirable range of strength.

It has been noted that when tube structures, manufactured as described above, are heated to above the lowest crystalline melting point of the PTFE, disorder begins to occur in the geometric order of the crystallites and the crystallinity decreases. This is accompanied by a concomitant increase in the amorphous content of the polymer. So formed amorphous regions within the crystalline structure greatly inhibit slippage along the crystalline axis of the crystallite and lock fibrils an crystallites so that they resist slippage under stress. Heat treatment may be considered to be, therefore, an amorphous locking process, the important aspect of which is an increase in the amorphous content of the treated structure. In fact, heat treatment above 327°C has been found to cause a two-fold increase in the strength of PTFE tubular structures.

The extrusion can be performed using dies of predetermined shape using principles known in the art. FIGURES 2 , and 2A and 2B show three orthogonal cross-sectional views of an exemplary die which can be used in the manufacture of a device of the present invention.

It should be understood that while several embodiments of the invention have been described in detail, the invention may be embodied in other specific forms without departing from the essential characteristics thereof. The present embodiments are therefore to be considered in all respects as illustrative and not restrictive, the scope of the invention being indicated by the appended claims rather than by the foregoing description.

## Claims

1. An implantable prosthetic device for sustained release of bioactive material into a fluid flow pathway of a patient comprising
a tubular body adapted for attachment to said fluid flow pathway having at least two interior lumina formed therein, namely a primary lumen (12) for accommodating fluid flow of said pathway therethrough, and a secondary lumen (12') extending adjacent said primary lumen (12), at least a portion of said secondary lumen (12') being separated from said primary lumen (12) by a membrane portion which is permeable to permit a bioactive material disposed in said secondary lumen (12') to permeate from said secondary lumen (12') through said membrane portion and into said primary lumen (12); **characterized in that**:
the body is extruded as a single tube with continuous wall defining both lumina, the secondary lumen being a space having an internal cross-dimension of at least 0.1mm.

2. The device according to claim 1, wherein said bioactive material is disposed in said secondary lumen (12') and is one of a therapeutic agent and a diagnostic agent.

3. The device as set forth in claim 1, wherein a said bioactive material is disposed in said secondary lumen and is a leachable strand of solid material.

4. The device of claim 1, further comprising a delivery means (20) for delivering said bioactive material from an external source into said secondary lumen (12').

5. The device of claim 4, wherein said delivery means comprises a mechanical or biologically activated pump (20).

6. The device of claim 1, wherein said secondary lumen (12') is filled with said bioactive material prior to implantation.

7. The device of claim 1 consisting substantially of a copolymeric material.

8. The device as claimed in claim 1 in which said body is formed from PTFE.

9. The device as claimed in claim 8 in which said body has been stretched to produce the body having predetermined porosity and flex quantities.

10. A method of making an implantable prosthetic device for sustained release of a bioactive material into a fluid flow pathway of a patient, said method comprising the steps of:
providing a die for a body adapted for attachment to said fluid flow pathway, said die including
(a) a peripheral support structure having an aperture for forming said body as a cross-sectionally continuous wall, said continuous wall defining at least two interior lumina, namely a primary lumen and a secondary lumen,
(b) a first solid die-piece located in said aperture for forming the primary lumen (12) to accommodate fluid flow pathway therethrough, and
(c) a second solid die-piece located adjacent and parallel to said first solid die-piece in said aperture to form the secondary lumen (12'), said second solid die-piece having an internal cross-dimension of at least 0.1mm; and
extruding said body along an extraction axis of the die, at least a portion of said secondary lumen (12') being separated from said primary lumen (12) by a membrane portion of said continuous wall (14) which is permeable to permit a material disposed in said secondary lumen (12') to permeate from said secondary lumen (12') through said membrane common portion and into said primary lumen (12).

11. The method of claim 10 in which the body is formed from PTFE.

12. The method of claim 11 including the step of stretching said single extruded body (10) to impart porosity throughout said body whereby the primary and secondary lumina form parallel chambers with controlled permeable communication therebetween within said body.

## Patentansprüche

1. Implantierbare prosthetische Vorrichtung zur verzögerten Freisetzung von biologisch aktivem Material in einen Fluid-Strömungsweg eines Patienten, umfassend:
einen zum Anbringen an diesen Fluid-Strömungsweg ausgelegten rohrförmigen Körper mit mindestens zwei darin ausgebildeten inneren Lumina, nämlich einem ersten Lumen (12) zur Aufnahme des Fluidstroms des hindurchgehenden Weges und einem zweiten Lumen (12'), das dem erstem Lumen (12) benachbart verläuft, wobei mindestens ein Teil des zweiten Lumens (12') von dem ersten Lumen (12) durch einen Membranteil getrennt ist, der durchlässig ist, damit es für biologisch wirksames, in dem zweiten Lumen (12') gelagertes Material möglich ist, von dem zweiten Lumen (12') durch den Membranteil in das erste Lumen (12) einzudringen;
**dadurch gekennzeichnet, daß**
der Körper als Einzelrohr mit kontinuierlicher Wand, die beide Lumina definiert, extrudiert ist, wobei das zweite Lumen ein Raum mit einer inneren Querabmessung von mindestens 0,1 mm ist.

2. Vorrichtung nach Anspruch 1, wobei das biologisch aktive Material in dem zweiten Lumen (12') gelagert und eines von einem therapeutischen Mittel und einem diagnostischen Mittel ist.

3. Vorrichtung nach Anspruch 1, wobei das biologisch aktive Material in dem zweiten Lumen enthalten und ein auslaugbarer Strang von festem Material ist.

4. Vorrichtung nach Anspruch 1, die außerdem ein Abgabemittel (20) zur Abgabe des biologisch aktiven Materials aus einer äußeren Quelle in das zweite Lumen (12') umfaßt.

5. Vorrichtung nach Anspruch 4, wobei das Abgabemittel eine mechanische oder biologisch aktivierte Pumpe (20) umfaßt.

6. Vorrichtung nach Anspruch 1, wobei das zweite Lumen (12') vor der Implantation mit dem biologisch aktiven Material befüllt wird.

7. Vorrichtung nach Anspruch 1 bestehend im wesentlichen aus einem copolymeren Material.

8. Vorrichtung nach Anspruch 1, wobei der Körper aus PTFE gebildet ist.

9. Vorrichtung nach Anspruch 8, wobei der Körper unter Erzeugung des Körpers mit festgelegten Porositäts- und Biegungseigenschaften gestreckt worden ist.

10. Verfahren zur Herstellung einer implantierbaren prosthetischen Vorrichtung zur verzögerten Abgabe eines biologisch aktiven Materials in einen Fluid-Strömungsweg eines Patienten, wobei das Verfahren die Schritte umfaßt:
Bereitstellen einer Hohlform für einen Körper, die zur Anbringung an den Fluid-Strömungsweg ausgelegt ist, wobei die Hohlform umfaßt:
a) eine periphere Trägerstruktur mit einer Öffnung zur Bildung des Körpers als eine im Querschnitt kontinuierliche Wand, wobei die kontinuierliche Wand mindestens zwei innere Lumina, nämlich ein erstes und ein zweites Lumen, definiert,
b) ein erstes festes Hohlform-Element, das zur Bildung des ersten Lumens (12) zur Aufnahme des hindurchgehenden Fluid-Strömungswegs in der Öffnung angeordnet ist, und
c) ein zweites festes Hohlform-Element, das benachbart und parallel zu dem ersten festen Hohlform-Element in der Öffnung unter Bildung des zweiten Lumens (12') angeordnet ist, wobei das zweite feste Hohlform-Element eine innere Querabmessung von mindestens 0,1 mm aufweist; und
Extrudieren des Körpers entlang einer Extraktionsachse der Hohlform, wobei mindestens ein Teil des zweiten Lumens (12') von dem ersten Lumen (12) durch einen Membranteil der kontinuierlichen Wand (14) getrennt ist, die durchlässig ist, um ein in dem zweiten Lumen (12') enthaltenes Material von dem zweiten Lumen (12') durch den gemeinsamen Membranteil und in das erste Lumen (12) eindringen zu lassen.

11. Verfahren nach Anspruch 10, wobei der Körper aus PTFE gebildet ist.

12. Verfahren nach Anspruch 11, das den Schritt des Streckens des einzelnen extrudierten Körpers (10) zur Vermittlung von Porosität auf den gesamten Körper umfaßt, wobei das erste und zweite Lumen im Körper parallele Kammern mit gesteuerter durchlässiger Kommunikation zwischen ihnen bilden.

## Revendications

1. Dispositif prothétique implantable pour la libération prolongée de matériau bioactif dans une voie d'écoulement de fluide d'un patient comprenant :
un corps tubulaire adapté pour être fixé à ladite voie d'écoulement de fluide ayant au moins deux lumières internes ci-dedans, plus précisément, une lumière primaire (12) pour permettre l'écoulement de fluide de ladite voie à travers celle-ci, et une lumière primaire (12') s'étendant de manière adjacente à ladite lumière principale (12), au moins une partie de ladite lumière secondaire (12') étant séparée de ladite lumière primaire (12) par une partie de membrane qui est perméable pour permettre à un matériau bioactif placé dans ladite lumière secondaire (12') de s'infiltrer à partir de ladite lumière secondaire (12') à travers ladite partie de membrane et dans ladite lumière primaire (12) ; **caractérisé en ce que** :
le corps est extrudé sous la forme d'un tube simple avec des parois continues définissant les deux lumières, la lumière secondaire étant un espace ayant une dimension transversale interne d'au moins 0,1 mm.

2. Dispositif selon la revendication 1, dans lequel ledit matériau bioactif est disposé dans ladite seconde lumière (12') et est l'un ou l'autre d'un agent thérapeutique et d'un agent de diagnostic.

3. Dispositif tel qu'exposé dans la revendication 1, dans lequel un dit matériau bioactif est disposé dans ladite lumière secondaire et est un fil lixiviable de matériau solide.

4. Dispositif selon la revendication 1, comprenant en outre des moyens de délivrance (20) pour la délivrance dudit matériau bioactif à partir d'une source externe dans ladite seconde lumière (12').

5. Dispositifs selon la revendication 4, dans lequel lesdits moyens de délivrance comprennent en outre une pompe mécanique ou une pompe activée d'une manière biologique.

6. Dispositif selon la revendication 1, dans lequel ladite lumière secondaire (12') est remplie dudit matériau bioactif avant l'implantation.

7. Dispositif selon la revendication 1 substantiellement formé d'un matériau copolymérique.

8. Dispositif tel que revendiqué dans la revendication 1 dans lequel ledit corps est formé à partir de PTFE.

9. Dispositif tel que revendiqué dans la revendication 8 dans lequel ledit corps a été étiré pour produire le corps ayant des quantités de flexibilité et de porosité prédéterminées.

10. Procédé de fabrication d'un dispositif prothétique implantable pour la libération prolongée d'un matériau bioactif dans une voie d'écoulement de fluide d'un patient, ledit procédé comprenant les étapes consistant à :
fournir un modèle pour un corps adapté pour être fixé à ladite voie d'écoulement de fluide, ledit modèle comprenant
(a) une structure de support périphérique ayant une ouverture pour former ledit corps sous la forme d'une paroi continue de manière transversale, ladite paroi continue définissant au moins deux lumières internes, plus précisément une lumière primaire et une lumière secondaire,
(b) une première pièce modèle solide située dans ladite ouverture pour former la lumière primaire (12) pour loger la voie d'écoulement de fluide à travers celle-ci, et
(c) une seconde pièce modèle solide située de manière adjacente et parallèle à ladite première pièce modèle solide dans ladite ouverture pour former ladite lumière secondaire (12'), ladite seconde pièce modèle solide ayant une dimension transversale interne d'au moins 0,1 mm ; et
extruder ledit corps le long d'un axe d'extraction du modèle, au moins une portion de ladite lumière secondaire (12') étant séparée de ladite lumière primaire (12) par une partie de membrane de ladite paroi continue (14) qui est perméable pour permettre à un matériau disposé dans ladite lumière secondaire (12') de s'infiltrer depuis ladite lumière secondaire (12') à travers ladite partie commune de membrane et dans ladite lumière primaire (12).

11. Procédé selon la revendication 10 dans lequel le corps est formé de PTFE.

12. Procédé selon la revendication 11 comprenant l'étape consistant à étirer ledit corps simple extrudé (10) pour impartir la porosité audit corps par lequel la lumière primaire et la lumière secondaire forment des chambres parallèles avec une communication perméable contrôlée entre ceci à l'intérieur dudits corps.
